# EUROPÄISCHE PATENTSCHRIFT

(11) **EP 2 214 725 B1**
(45) Veröffentlichungstag und Bekanntmachung des Hinweises auf die Patenterteilung: **20.02.2013**
(21) Anmeldenummer: 08848853.1
(22) Anmeldetag: 13.11.2008
(51) Int. Cl.: A61L 2/24

(54) **VERFAHREN, VORRICHTUNG UND COMPUTERPROGRAMM ZUM VISUALISIEREN DES TROCKNUNGSVORGANGS VON ENDOSKOPEN**
METHOD, DEVICE AND COMPUTER PROGRAM FOR VISUAL DISPLAY OF THE DRYING OF ENDOSCOPES
PROCÉDÉ, DISPOSITIF ET PROGRAMME INFORMATIQUE POUR VISUALISER LE PROCESSUS DE SÉCHAGE D'ENDOSCOPES

(30) Priorität: 13.11.2007 DE 102007054032
(43) Veröffentlichungstag der Anmeldung: 11.08.2010
(73) Patentinhaber: Endo-Technik Wolfgang Griesat Gmbh, 42697 Solingen (DE)
(72) Erfinder: TRANCHINA, Gioacchino, 42697 Solingen (DE)
(74) Vertreter: Nätebusch, Roderich
(86) Internationale Anmeldenummer: PCT/DE2008/001876
(87) Internationale Veröffentlichungsnummer: WO 2009/062490

(56) Entgegenhaltungen:
- WO-A-93/06948
- WO-A-2005/003273
- US-A1- 2005 196 314

## Beschreibung

Die vorliegende Erfindung bezieht sich auf ein Verfahren, eine Vorrichtung und ein Computerprogramm zum Visualisieren von Trocknungsvorgängen von in einem Trockenschrank untergebrachten Endoskopen nach deren Reinigung und/oder Desinfektion. Die Erfindung betrifft ferner ein Computerprogramm zum Betreiben einer das Verfahren gemäß der Erfindung ausführenden Vorrichtung.

Es ist bereits eine Vorrichtung zum Reinigen, Sterilisieren, Transportieren und Lagern von medizinischen Geräten, insbesondere von Endoskopen bekannt (DE 197 21 538 A1), bei der die medizinischen Geräte und insbesondere die Endoskope zur Reinigung, zur Sterilisierung, zum Transportieren und zum Lagern stets in ein und demselben verschlossenen Behälter bleiben. Eine Visualisierung des Trocknungsvorgangs der medizinischen Geräte und insbesondere von Endoskopen ist dabei allerdings nicht vorgesehen.

Im Zusammenhang mit einem Prozess zur Aufbereitung eines medizinischen Gerätes ist außerdem ein Überwachungs- und Steuersystem bekannt (US 2005/196314 A1), mit dem kritische Schritte dieses Prozesses überwacht werden, der von einem Hardware-Controller unter Steuerung eines Computers mit einem herkömmlichen Betriebssystem ausgeführt wird. Dieses bekannte Überwachungs- und Steuersystem weist eine Master-Steuerung auf, die zwischen dem Computer und dem Hardware-Controller angeschlossen ist und mittels der die erwähnten kritischen Schritte überwacht werden können. Dadurch kann bei dem bekannten Überwachungs- und Steuersystem festgestellt werden, ob der jeweilige kritische Schritt ausgeführt und ob er auch richtig ausgeführt wird. Die Steuerung des Hardware-Controllers kann dabei dem Computer entzogen werden, falls in einem kritischen Schritt ein Fehler beobachtet wird.

Es ist ferner ein Verfahren zur Herstellung von Perkohlensäure für die Behandlung/Reinigung von Substraten oder medizinischen Geräten bekannt (WO2005/003273 A). Dabei ist zwar auch eine Echtzeit-Überwachung von Behandlungsprozessen angegeben. Diese Überwachung läuft jedoch ohne eine Visualisierung ab.

Es besteht jedoch zuweilen der Wunsch, eine solche Visualisierung des Trocknungsvorgangs der medizinischen Geräte und insbesondere von Endoskopen den Benutzern der betreffenden Geräte und insbesondere der Endoskope zur Verfügung zu stellen, mitunter auch unter dem Gesichtspunkt einer Protokollierung des jeweiligen Trocknungsvorgangs.

Der Erfindung liegt daher die Aufgabe zu Grunde, einen Weg zu zeigen, wie bei einem Verfahren und bei einer Vorrichtung der eingangs genannten Art auf relativ einfache Weise eine Visualisierung von Trocknungsvorgängen von in einem Trockenschrank untergebrachten Endoskopen entsprechend deren Reinigung bzw. Desinfektion erfolgen kann.

Gelöst wird die vorstehend aufgezeigte Aufgabe zum einen durch ein Verfahren zur Visualisierung von Trocknungsvorgängen bei einer Mehrzahl von in einem Trockenschrank untergebrachten Endoskopen nach deren Reinigung und/oder Desinfektion mittels einer Visualisierungsvorrichtung,
wobei mittels der Visualisierungsvorrichtung in einer Anzeige-Bildschirmmaske schematische Endoskopdarstellungen der zu trocknenden Endoskope angezeigt werden
und wobei die schematischen Endoskopdarstellungen der zu trocknenden Endoskope in der Visualisierungsvorrichtung bei fortschreitender Trocknung der betreffenden Endoskope in einer Weise verändert werden, insbesondere durch farbliche Füllung und/oder eine Formveränderung, welche vom Ausgangszustand der Trocknung der Endoskope verschieden ist.

Hierdurch ergibt sich der Vorteil, dass auf relativ anschauliche Weise das Fortschreiten von Trocknungsvorgängen von in dem Trocknungsschrank zum Trocknen unter gebrachten Endoskopen individuell visualisiert werden kann.

Zweckmäßigerweise wird die Anzeige-Bildschirmmaske als Hintergrundmaske oder als Bildschirmschoner in einer Anzeigeeinrichtung benutzt. Dies ermöglicht, die Anzeigeeinrichtung außer zur Visualisierung des fortschreitenden Trocknungsvorgangs von Endoskopen auch zur Anzeige von beliebigen anderen Vorgängen nutzen zu können.

Vorzugsweise werden das Trocknen betreffende Parameter als Einstellgrößen, insbesondere als Trocknungstemperatur und/oder Trocknungsluftgeschwindigkeit, mittels den einzelnen Endoskopen bzw. deren zugehörigen schematischen Endoskopdarstellungen zugehöriger Software-Tasten individuell eingestellt, die insbesondere in einer gesonderten Bildschirmmaske dargestellt werden. Hierdurch ergibt sich der Vorteil, dass zwischen den im Trocknungsschrank zum Trocknen untergebrachten Endoskopen und den erwähnten Bildschirmmasken gewissermaßen ein interaktiver Datenaustausch ermöglicht ist, der für die erwähnte Visualisierung besonders günstig ist.

Gemäß zweckmäßiger Weiterbildung der Erfindung werden den einzelnen Endoskopdarstellungen gegebenenfalls in einer weiteren Bildschirmmaske Anzeigebereiche zugeordnet, in denen Werte der durch das jeweilige Endoskop hindurchgeleitete Trocknungsluft pro Zeiteinheit und der Resttrocknungszeit angezeigt werden. Dies bringt den Vorteil einer quantitativen, detaillierteren Visualisierung von Trocknungsparametem mit sich.

Vorzugsweise wird durch ein tastenbetätigtes Auswahlmenü der jeweiligen Endoskopdarstellung eine dem entsprechenden Endoskop zugehörige Geräte- und/oder Typenbezeichnung zugeordnet und angezeigt. Dadurch ergibt sich der Vorteil, dass für den Betreiber des Trockenschrankes wichtige Informationsdaten bezüglich der zu trocknenden Endoskope zusätzlich visualisiert werden können.

Zweckmäßigerweise werden sämtliche Darstellungen bzw. Anzeigen durch Anklicken wenigstens einer Software-Taste in einer Speichervorrichtung für eine Protokollierung der Trocknungsvorgänge sowie für die Erstellung von Diagrammen gespeichert. Auf diese Weise stehen erforderliche Protokollierungsdaten auch nach Ablauf der Trocknungsvorgänge noch für unterschiedliche Auswertungen zur Verfügung.

Zum anderen wird die oben aufgezeigte Aufgabe durch eine Vorrichtung zur Visualisierung von Trocknungsvorgängen bei einer Mehrzahl von in einem

Trockenschrank untergebrachten Endoskopen nach deren Reinigung und/oder Desinfektion mittels einer Visualisierungsvorrichtung, umfassend Mittel zur Ausführung des Verfahrens nach Anspruch 1,
wobei in der Visualisierungsvorrichtung eine Anzeige-Bildschirmmaske mit schematischen Endoskopdarstellungen der zu trocknenden Endoskope anzeigbar ist und wobei die schematischen Endoskopdarstellungen der zu trocknenden Endoskope in der Anzeige-Bildschirmmaske bei der fortschreitenden Trocknung der betreffenden Endoskope in einer Weise verändert sind, insbesondere durch farbliche Füllung und/oder eine Formveränderung, welche vom Ausgangszustand der Trocknung der Endoskope verschieden ist.

Hierdurch ergibt sich der Vorteil, dass sich mit relativ geringem Aufwand in anschaulicher Weise das Fortschreiten von Trocknungsvorgängen von in dem Trocknungsschrank zum Trocknen untergebrachten Endoskopen Individuell visuallsleren:lässt.

Zweckmäßigerweise ist die Anzeige-Bildschirmmaske als Hintergrundmaske oder als Bildschirmschoner In einer Anzeigeeinrichtung nutzbar. Dies erlaubt es in vorteilhafter Weise, die Anzeigeeinrichtung außer zur Visualisierung des Fortschreitens von Trocknungsvorgängen von Endoskopen auch zur Anzeige von beliebigen anderen Vorgängen nutzen zu können.

Entsprechend einer zweckmäßigen Weiterbildung der Vorrichtung gemäß der. Erfindung sind das Trocknen betreffende Parameter als Einstellgrößen, insbesondere als Trocknungstemperatur und/oder Trocknungsluftgeschwindigkeit, mittels den einzelnen Endoskopen bzw. deren zugehörigen schematischen Endoskopdarstellungen zugehöriger Software-Tasten individuell einstellbar, die insbesondere in einer gesonderten Bildschirmmaske enthalten sind. Dies bringt den Vorteil mit sich, dass zwischen den im Trockenschrank zum Trocknen untergebrachten Endoskopen und den erwähnten Bildschirmmasken gewissermaßen ein interaktiver Datenaustausch ermöglicht ist, der für die erwähnte Visualisierung besonders günstig ist. Vorzugsweise sind den einzelnen Endoskopdarstellungen gegebenenfalls in einer weiteren Bildschirmmaske Anzeigebereiche zugeordnet, in denen Werte der durch das jeweilige Endoskop hindurchgeleiteten Trocknungsluft pro Zeiteinheit und/oder die Resttrocknungszeit anzeigbar sind. Dadurch ergibt sich der Vorteil einer quantitativen, detaillierteren Visualisierungsmöglichkeit von Trocknungsparametern.

Zweckmäßigerweise lässt sich durch ein tastenbetätigtes Auswahlmenü der jeweiligen Endoskopdarstellung eine dem entsprechenden Endoskop zugehörige Geräte- und/oder Typenbezeichnung zuordnen und anzeigen. Dadurch können für den Betreiber des Trockenschrankes wichtige Informationsdaten bezüglich der zu trocknenden Endoskope zusätzlich visualisiert werden.

Vorzugsweise ist eine Speichervorrichtung vorgesehen, in der sämtliche Darstellungen und/oder Anzeigewerte durch Anklicken wenigstens einer Software-Taste für eine Protokollierung der Trocknungsvorgänge sowie für die Erstellung von Diagrammen speicherbar sind. Hierdurch ergibt sich der Vorteil, dass mit relativ geringem Aufwand erforderliche Protokollierungsdaten auch nach Ablauf der Trocknungsvorgänge noch für unterschiedliche Auswertungen zur Verfügung stehen.

Die vorliegende Erfindung betrifft außerdem ein Computerprogramm zum Betreiben einer das Verfahren gemäß der Erfindung ausführenden Vorrichtung. Dieses Computerprogramm kann auf einem Speichermedium wie einer CD-ROM oder einer CD enthalten sein, von der Programmdaten lesbar und auf der Protokollierungsdaten durch Schreibvorgänge speicherbar sind.

Anhand von Zeichnungen werden nachstehend Ausführungsbeispiele der vorliegenden Erfindung näher erläutert.
- Fig. 1: zeigt eine erste Bildschirmmaske eines Visualisierungsprogramms.
- Fig. 2: zeigt eine zweite Bildschirmmaske des Visualisierungsprogramms.
- Fig. 3: zeigt eine dritte Bildschirmmaske des Visualisierungsprogramms, die bei Anklicken einer in der Bildschirmmaske gemäß Fig. 2 vorgesehenen Softwaretaste "Serviceparameter" erscheint.
- Fig. 4: zeigt eine vierte Bildschirmmaske des Visualisierungsprogramms, die nach positiven Eingaben in der dritten Bildschirmmaske erscheint.
- Fig. 5: zeigt eine fünfte Bildschirmmaske, die als Untermenü aus der vierten Bildschirmmaske aufrufbar ist.
- Fig. 6: zeigt eine sechste Bildschirmmaske, die eine Kennwortabfrage betrifft, welche vor Eintritt in eine Benutzerverwaltung gemäß der zweiten Bildschirmmaske erscheint.
- Fig. 7: zeigt eine siebte Bildschirmmaske, welche eine Benutzerverwaltung betrifft.
- Fig. 8: zeigt eine achte Bildschirmmaske, die beim Anlegen eines neuen Benutzemamens erscheint.
- Fig. 9: zeigt die bereits in Fig. 7 gezeigte siebte Bildschirmmaske, allerdings mit geänderten Benutzemamen.
- Fig. 10: zeigt die in Fig. 9 dargestellte Bildschirmmaske mit zusätzlich geänderten Benutzemamen.
- Fig. 11: zeigt eine neunte Bildschirmmaske, die eine Kennwortabfrage betrifft, welche vor Eintritt in eine Endoskopverwaltung gemäß der zweiten Bildschirmmaske erscheint.
- Fig. 12: zeigt eine zehnte Bildschirmmaske, welche die Endoskopverwaltung betrifft.
- Fig. 13: zeigt eine elfte Bildschirmmaske, die als Untermenü aus der zehnten Bildschirmmaske aufrufbar ist.
- Fig. 14: zeigt die zehnte Bildschirmmaske mit einem neuen Eintrag.

Bevor auf die Zeichnungen näher eingegangen wird, sei angemerkt, dass das Visualisierungsprogramm zur Ausführung des Verfahrens gemäß der Erfindung auf einem normalen Personalcomputer ablaufen kann und dass die einzelnen Bildschirmmasken auf einem mit diesem verbundenen Monitor angezeigt werden können. Der betreffende Personalcomputer weist zumindest eine Tastatur und eine Maus als Eingabeeinrichtungen auf. Ein in dem betreffenden Personalcomputer enthaltener Speicher, zum Beispiel dessen Festplatte, kann zur Abspeicherung der die einzelnen Trocknungsvorgänge betreffenden Parameter bzw. Daten genutzt werden. Diese über die Zeit aufgezeichneten Parameter können dann zum Erstellen von Diagrammen herangezogen werden, mit denen eine Protokollierung der Trocknungsvorgänge ermöglicht ist.

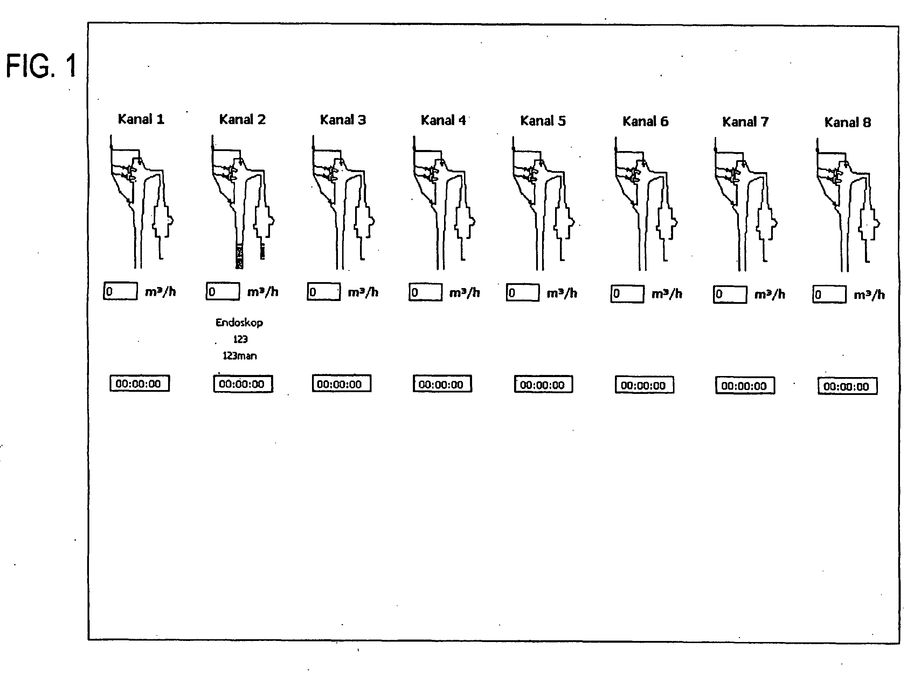

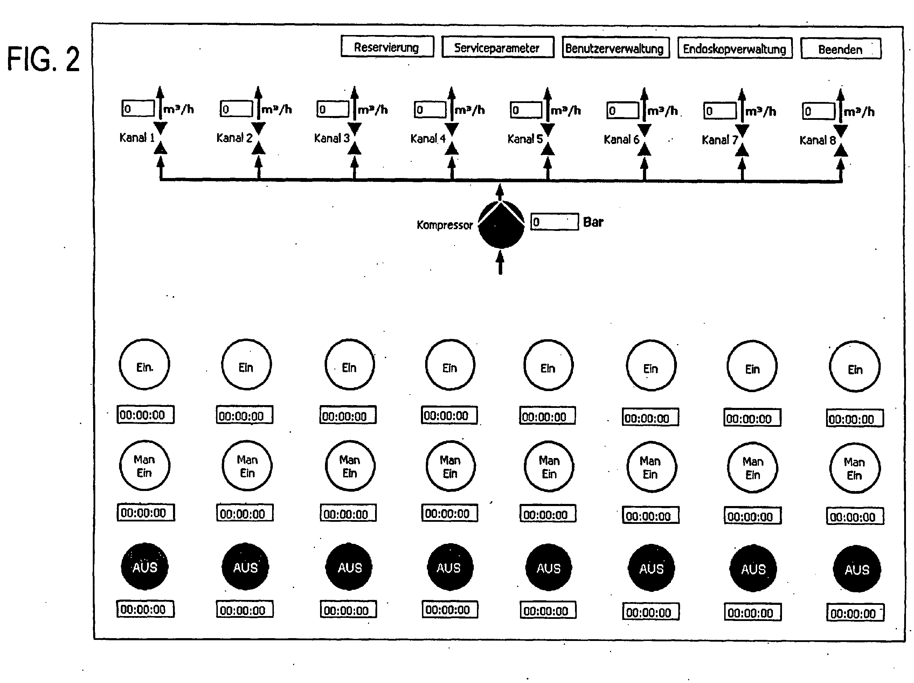

Von den in den Zeichnungen dargestellten Bildschirmmasken dient die in Fig. 1 gezeigte erste Bildschirmmaske vorzugsweise als Hintergrund- bzw. Bildschirmschonermaske des eigentlichen Visualisierungsprogramms, welches mit der in Fig. 2 gezeigten zweiten Bildschirmmaske im Detail beginnt. Bei Nutzung der Bildschirmmaske gemäß Fig. 1 als Hintergrundmaske tritt diese Bildschirmmaske ständig auf einem Anzeigeschirm auf. Wird die Bildschirmmaske gemäß Fig. 1 als Bildschirmschonermaske genutzt, so erscheint sie auf dem betreffenden Anzeigeschirm automatisch, wenn während einer festgelegten Zeitspanne keine Eingaben in der Vorrichtung bzw. dem Personalcomputer erfolgt sind, auf der bzw. dem das Visualisierungsprogramm abläuft.

Die in Fig. 1 gezeigte erste Bildschirmmaske zeigt beispielsweise in acht Kanälen, die mit Kanal 1 bis Kanal 8 bezeichnet sind, einzelnen Endoskopen im zugehörigen (nicht dargestellten Trockenschrank) zugeordnete schematische Endoskopdarstellungen, welche das Fortschreiten der Trocknung der entsprechenden Endoskope in einer Weise veranschaulichen, insbesondere durch ihre farbliche Füllung und/oder durch eine Formveränderung, die vom Ausgangszustand der Trocknung verschieden ist. So lässt die dem Kanal 2 zugehörige Endoskopdarstellung bereits eine solche Farb- und Formveränderung erkennen. Jedem der Kanäle 1 bis 8 sind überdies zwei Anzeigefelder zugehörig, in denen wichtige Informationen bezüglich des jeweiligen kanalindividuellen Trocknungsvorgangs anzeigbar sind, wie zum Beispiel die Druckluftmenge in m³/h (im oberen Anzeigefeld) und die Restdauer des jeweiligen Trocknungsvorgangs (im unteren Anzeigefeld) im Anzeigeformat hh, mm, ss - also im zweistelligen Format Stunden, Minuten und Sekunden.

Von der in Fig. 1 gezeigten ersten Bildschirmmaske gelangt man zu der in Fig. 2 gezeigten zweiten Bildschirmmaske durch entsprechende Cursorbewegung und einer Betätigung einer Maustaste der erwähnten Maus, beispielsweise unter Anklicken irgendeines beliebigen Bereiches in der Bildschirmmaske gemäß Fig. 1, der nicht mit einem Anzeigeelement belegt ist, oder eines für diesen Wechsel dort vorgesehenen besonderen Anklickbereiches.

Wie aus Fig. 2 ersichtlich ist kann von dieser Bildschirmmaske ausgehend der Trocknungsprozess bezüglich der in einem (hier nicht dargestellten) Trockenschrank untergebrachten Endoskope zum einen gesteuert und überwacht und zum anderen hinsichtlich seines Fortschreitens visualisiert werden. Dem Trockenschrank wird in bekannter Weise von einem Luftkompressor Druckluft zum Hindurchleiten durch die verschiedenen Lumen der in ihm zum Trocknen untergebrachten Endoskope zugeführt. Bezüglich dieser Druckluft kann die Luftmenge pro Zeiteinheit, der Luftdruck und gegebenenfalls die Temperatur innerhalb vorgegebener Grenzen softwaremäßig eingestellt und mittels entsprechender Sensoren individuell für jedes der in dem Trockenschrank enthaltenen Endoskope ermittelt werden. Derartige Messwerte und der zeitlich fortschreitende Trocknungsvorgang werden gemäß der vorliegenden Erfindung visualisiert.

Die Steuerung bzw. Einstellung der verschiedenen Vorgänge erfolgt in der Bildschirmmaske gemäß Fig. 2 durch Betätigen bzw. Anklicken von Software-Tasten mittels einer Maustaste der erwähnten Maus - im folgenden jeweils nur kurz als Anklicken bezeichnet. Diese Software-Tasten sind im oberen Teil der Fig. 2 mit "Reservierung", "Serviceparameter", "Benutzerverwaltung", "Endoskopverwaltung" und "Beenden" bezeichnet, und sie sind im unteren Teil der Fig. 2 durch den acht Kanälen K1 bis K8, die im Zusammenhang mit Fig. 1 erwähnt worden sind, individuell zugeordnete Steuertasten "Ein", "Man Ein" und "AUS" angedeutet. Die Funktionen der Software-Tasten "Ein" und "Man Ein" sind unterschiedlich. So kann durch Anklicken einer Software-Taste "Ein" der Trocknungsvorgang eines Endoskops im zugehörigen Kanal gestartet werden, nachdem zuvor die für das Trocknen erforderlichen Parameter eingegeben worden sind; durch Anklicken einer Software-Taste "Man Ein" kann beispielsweise ein unterbrochener Trocknungsvorgang nach einer erfolgten Veränderung von Trocknungsparametem an einer der Veränderung entsprechenden Stelle des Trocknungsprogramms oder an der der Unterbrechung im zuerst abgelaufenen Trocknungsvorgang fortgesetzt werden. Gegebenenfalls können die Software-Tasten "Man Ein" auch entfallen. Durch Anklicken einer Software-Taste "AUS" kann ein Trocknungsvorgang eines Endoskops in dem zugehörigen Kanal ausgeschaltet werden.

Den Darstellungen der einzelnen Kanäle 1 bis 8 sind in der Bildschirmmaske gemäß Fig. 2 entsprechende kanalindividuellen Anzeigefelder zugehörig, in denen die kanalindividuellen Druckluftmengen in m³/h der an die zugehörigen Endoskope im Trockenschrank abgegebenen Trocknungsluft anzeigbar sind.

Den erwähnten Steuertasten "Ein", "Man Ein" und "AUS" sind Zeitanzeigefelder zugehörig, in denen Zeitangaben im zweistelligen Anzeigeformat hh, mm, ss - also im Format Stunden, Minuten und Sekunden - für eine Trocknungszeit ("Ein"), eine abgelaufene Zeit seit Beginn des Trocknungsvorgangs eines Endoskops im zugehörigen Kanal ("Man Ein") und eine Resttrocknungsdauer ("AUS") kanalindividuell angezeigt werden.

Außerdem ist in Fig. 2 im oberen Bereich neben einem mit Kompressor bezeichneten Symbol noch ein weiteres, mit "Bar" bezeichnetes Anzeigefeld vorgesehen, in welchem der Druck, beispielsweise in mBar, der Druckluft angezeigt wird, die ein mit dem erwähnten Trockenschrank verbundener Kompressor liefert.

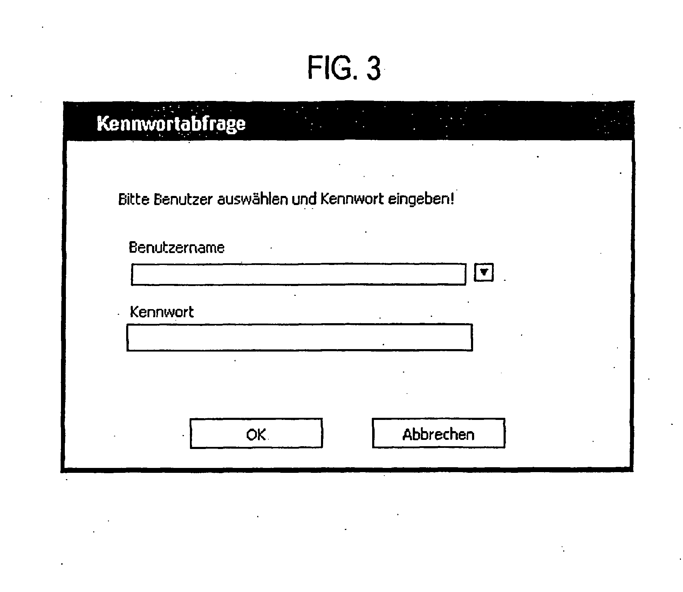

Nach Anklicken der Software-Taste "Serviceparameter" in der Bildschirmmaske gemäß Fig. 2 öffnet sich die in Fig. 3 dargestellte Bildschirmmaske, die in zwei Eingabefeldern die Eingabe eines Benutzernamens bzw. eines Kennwortes (Passwortes) erfordert. Die zugelassenen Benutzernamen und das Kennwort sind vorab durch einen Systemadministrator festgelegt und im Visualisierungsprogramm abgespeichert worden. Rechts neben dem Eingabefeld "Benutzername" befindet sich ein Pulldown-Menü, durch dessen Anklicken die Liste der zugelassenen Benutzernamen aufgerufen und einer dieser Namen ausgewählt werden kann.

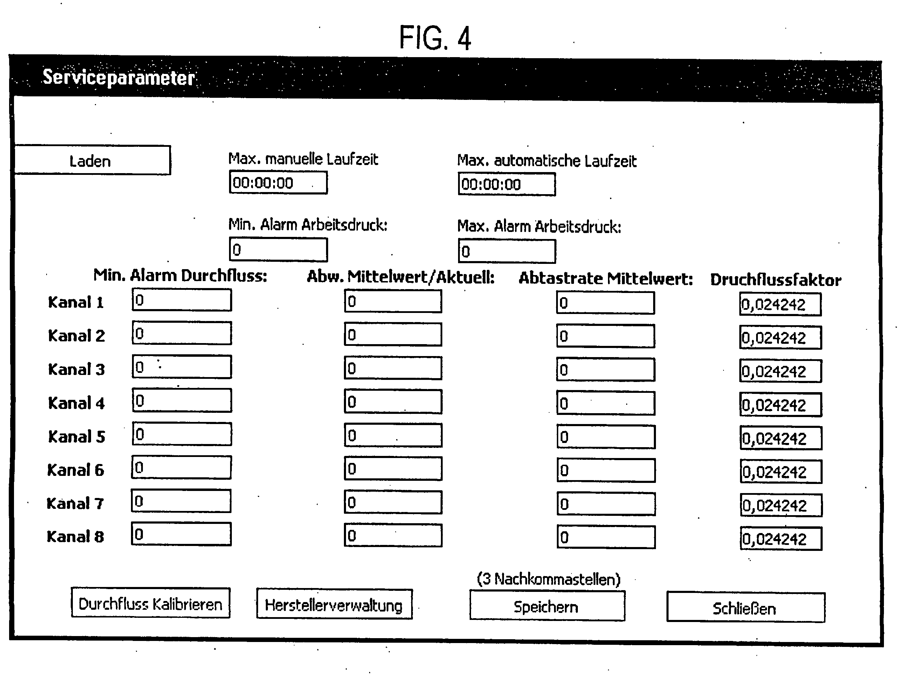

Nach erfolgreichen Eingaben in die Eingabefelder und Anklicken der Software-Taste "0K" gemäß Fig. 3 wird schließlich die Bildschirmmaske "Serviceparameter" gemäß Fig. 4 geöffnet. In den einzelnen Anzeigefeldern dieser Bildschirmmaske können dann für die zu trocknenden Endoskope entsprechend den Kanälen 1 bis 8 erforderliche Druckwerte und Zeitwerte sowie Druckluft-Durchtlussmengen eingetragen oder aus vorhandenen gespeicherten Werten durch Betätigen der Software-Taste "Laden" abgerufen und eingetragen werden.

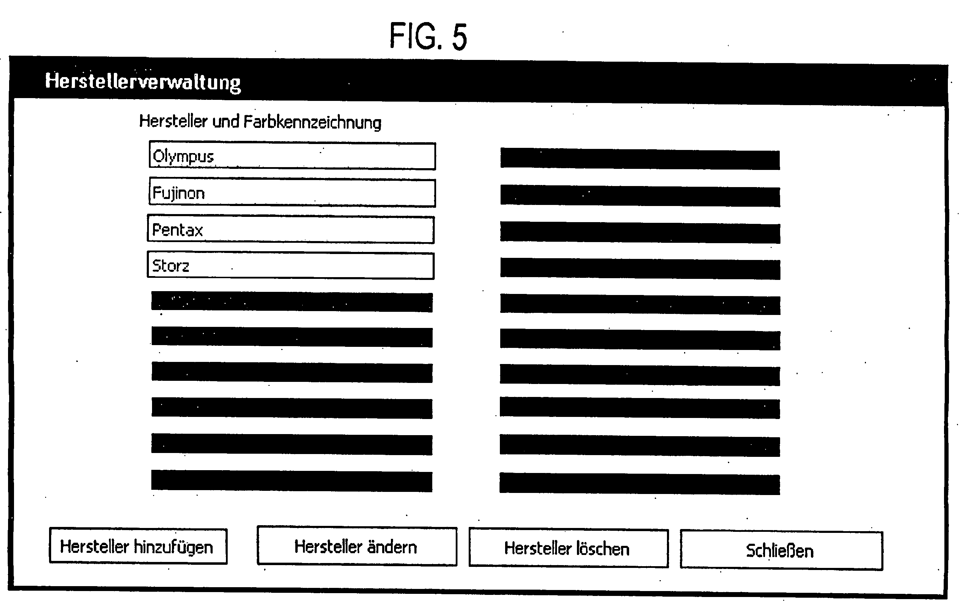

Die so eingestellten Werte können dann durch Anklicken der im unteren Bereich der Bildschirmmaske gemäß Fig. 4 enthaltenen Software-Taste "Speichern" gespeichert werden. Durch Anklicken der Software-Taste "Durchfluss kalibrieren" lässt sich der kanalindividuelle Druckluftdurchfluss einstellen. Durch Anklicken der Software-Taste "Schließen" gelangt man wieder zur Bildschirmmaske gemäß Fig. 2. Durch Anklicken der Software-Taste "Herstellerverwaltung" gelangt man allerdings zur Bildschirmmaske gemäß Fig. 5, und zwar ohne die Forderung nach zusätzlicher Eingabe eines Benutzemamens und eines Kennwortes.

In der ein Untermenü der Bildschirmmaske gemäß Fig. 4 darstellenden Bildschirmmaske gemäß Fig. 5 besteht die Möglichkeit, durch Eingabe in die im oberen Teil vorgesehenen Eintragfelder die Namen von Endoskopherstellem einzutragen und durch Anklicken der im unteren Teil vorgesehenen Software-Tasten "Hersteller hinzufügen", "Hersteller ändern" oder "Hersteller löschen" diesen Angaben entsprechende Vorgänge im Visualisierungsprogramm auszulösen. Durch Anklicken der im unteren Teil vorgesehenen Software-Taste "Schließen" gelangt man wieder zu der Bildschirmmaske gemäß Fig. 2.

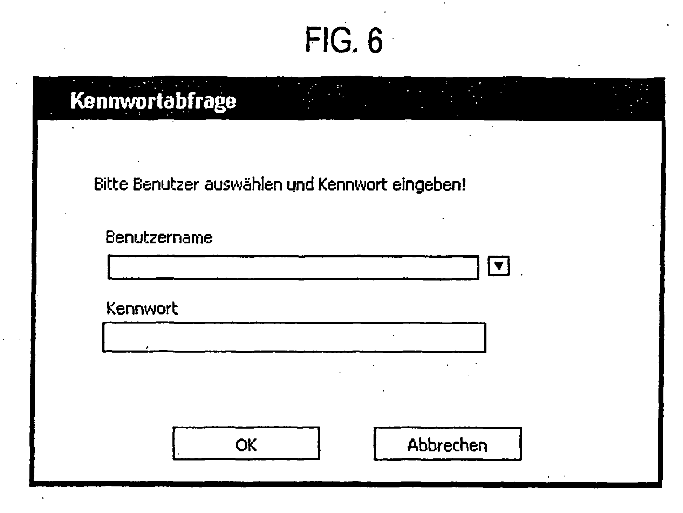

Wird in der Bildschirmmaske gemäß Fig. 2 die Software-Taste "Benutzerverwaltung" angeklickt, so gelangt man zunächst zu der Bildschirmmaske gemäß Fig. 6, die der Bildschirmmaske gemäß Fig. 3 entspricht. Zu der Bildschirmmaske gemäß Fig. 6 treffen daher dieselben Erläuterungen zu, die zur Bildschirmmaske gemäß Fig. 3 gegeben worden sind.

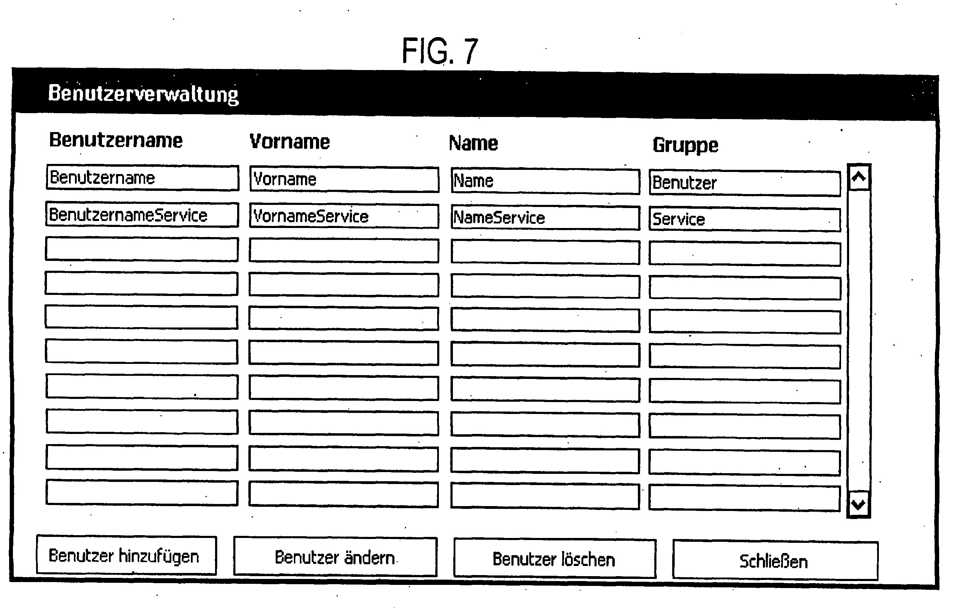

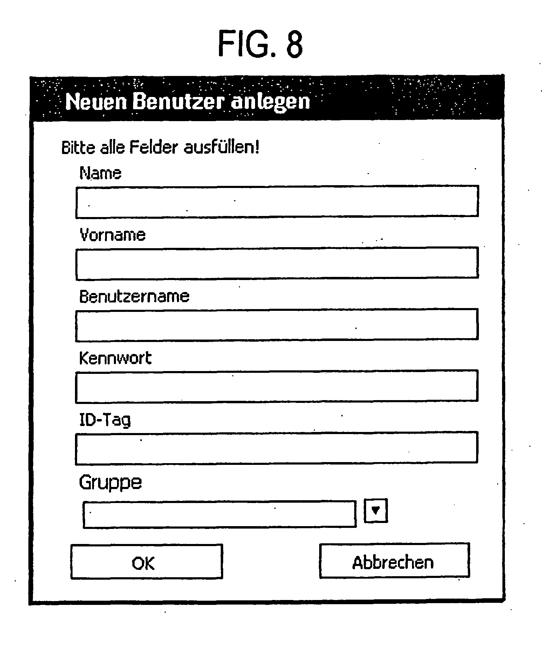

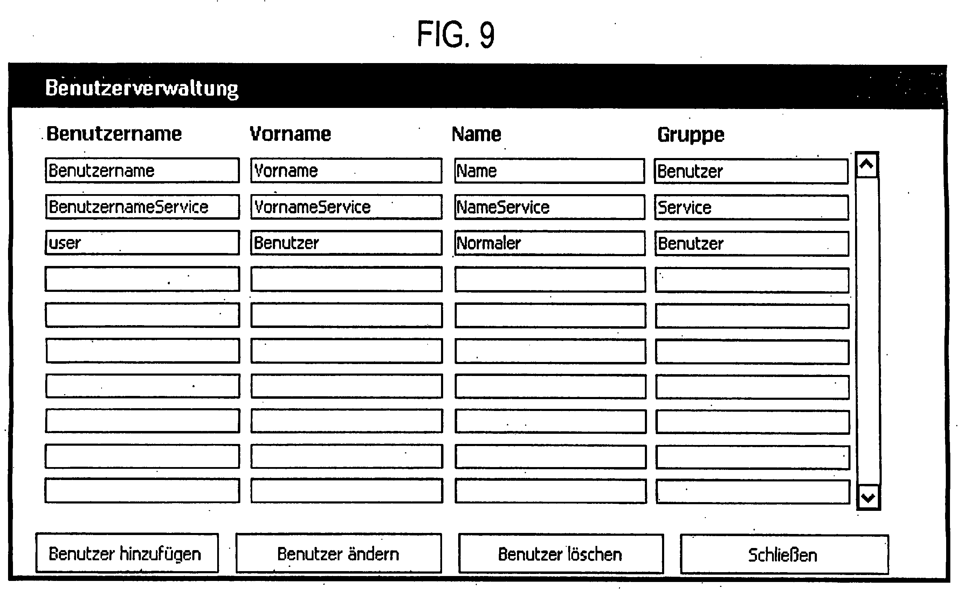

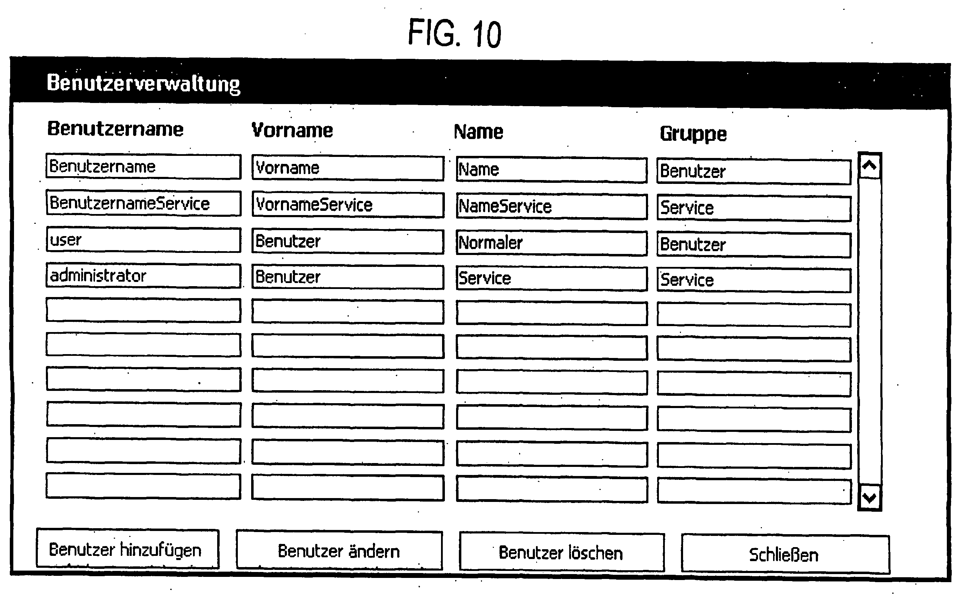

Nach erfolgreichen Eingaben in die Eingabefelder und Anklicken der Software-Taste "OK" gemäß Fig. 6 wird die Bildschirmmaske "Benutzerverwaltung" gemäß Fig. 7 geöffnet. Hier treffen die entsprechenden Erläuterungen zu, die zur Bildschirmmaske gemäß Fig. 5 gegeben wurden. Beim Anklicken der im unteren Teil vorhandenen Software-Taste "Benutzer hinzufügen" gelangt man zu der Bildschirmmaske gemäß Fig. 8, in die die entsprechenden Angaben eingetragen werden können. Nach Anklicken der Software-Taste "OK" gelangt man dann zur Bildschirmmaske gemäß Fig. 9 oder gemäß Fig. 10, in denen gegenüber der Bildschirmmaske gemäß Fig. 7 neue Benutzernamen angezeigt werden. Durch Anklicken der im unteren Teil vorgesehenen Software-Taste "Schließen" gelangt man aus den Bildschirmmasken gemäß Fig. 7, 9 und 10 jeweils wieder zu der Bildschirmmaske gemäß Fig. 2 zurück.

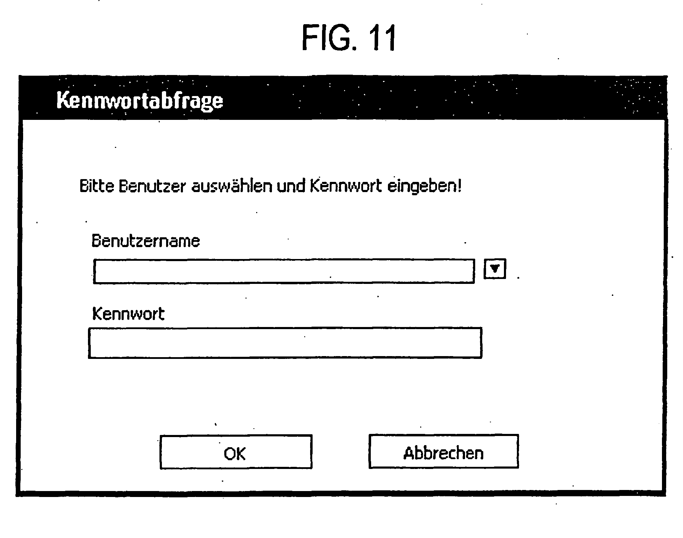

Nach Anklicken der Software-Taste "Endoskopverwaltung" in der Bildschirmmaske gemäß Fig. 2 öffnet sich die in Fig. 11 dargestellte Bildschirmmaske, die der Bildschirmmaske gemäß Fig. 3 entspricht. Zu der Bildschirmmaske gemäß Fig. 11 treffen daher dieselben Erläuterungen zu, die zur Bildschirmmaske gemäß Fig. 3 gegeben worden sind.

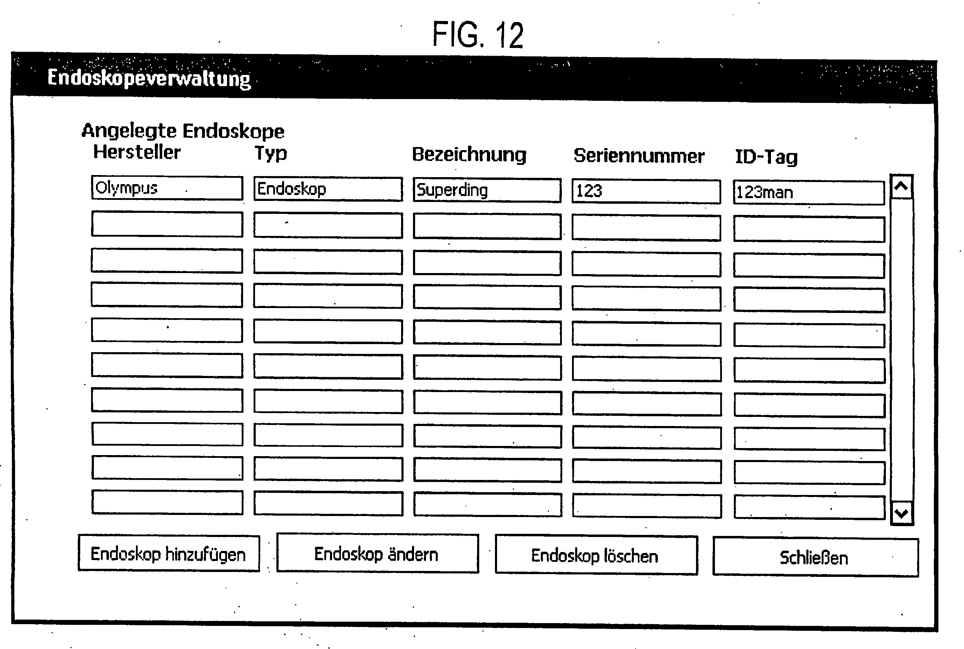

Nach erfolgreichen Eingaben in die Eingabefelder und Anklicken der Software-Taste "OK" gemäß Fig. 11 wird die Bildschirmmaske "Endoskopverwaltung" gemäß Fig. 12 geöffnet. In diese Bildschirmmaske können für die in dem Trockenschrank zu trocknenden Endoskope verschiedene Angaben eingetragen werden, wie der Herstellername, der Endoskoptyp, die Endoskopbezeichnung, die Seriennummer und ein ID-Tag bzw. -Kennzeichen.

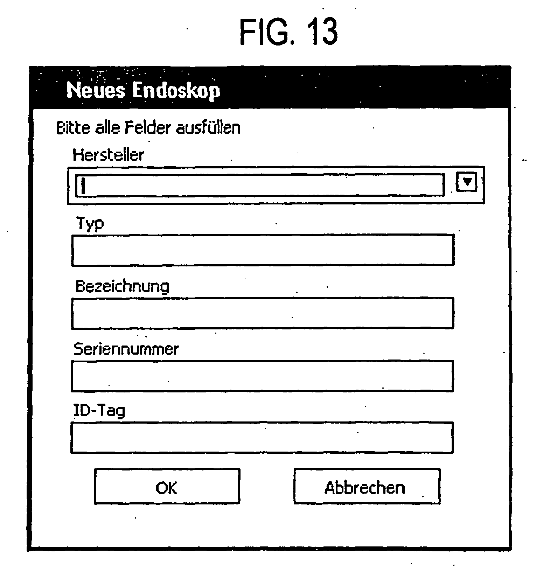

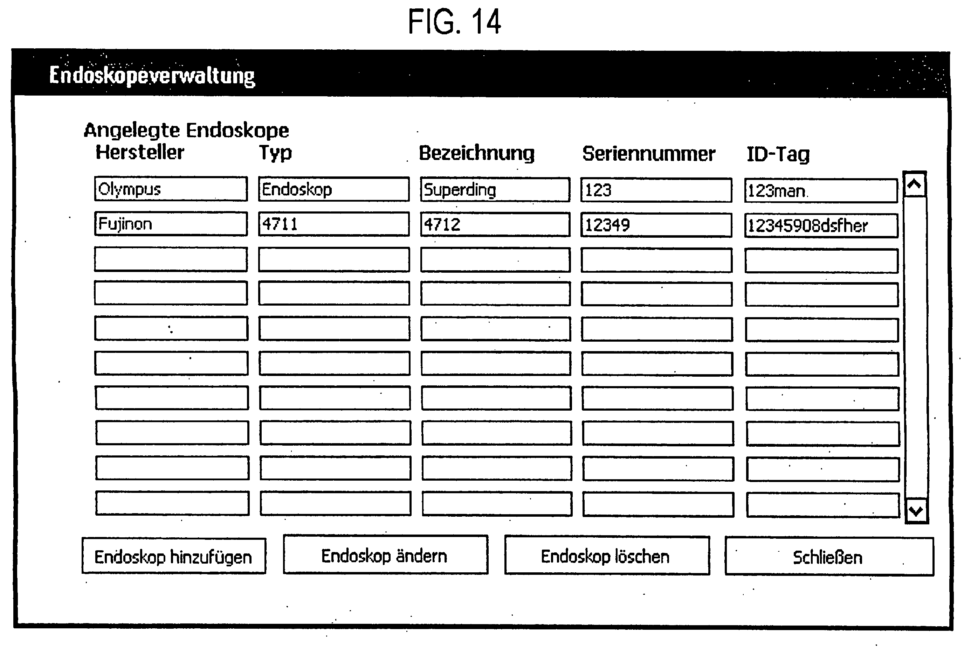

Durch Anklicken der im unteren Teil dieser Bildschirmmaske vorhandenen Software-Taste "Endoskop hinzufügen" gelangt man zu der Bildschirmmaske gemäß Fig. 13, in die die entsprechenden Angaben eingetragen werden können. Nach Anklicken der Software-Taste "OK" in dieser Bildschirmmaske gelangt man dann zur Bildschirmmaske gemäß Fig. 14, die im Vergleich zur Bildschirmmaske gemäß Fig. 12 die nunmehr veränderten Verhältnisse in der Endoskopverwaltung zeigt. Nach Anklicken der Software-Taste "Schließen" in der Bildschirmmaske gemäß Fig. 12 oder Fig. 14 gelangt man wieder zu der Bildschirmmaske gemäß Fig. 2.

Durch Anklicken der im oberen Teil der Bildschirmmaske gemäß Fig. 2 dargestellten Software-Taste "Beenden" läßt sich das Visualisierungsprogramm beenden. Zugleich damit wird der Trocknungsprozess sämtlicher im erwähnten Trockenschrank befindlicher Endoskope gestoppt.

Im oberen Teil der Bildschirmmaske gemäß Fig. 2 ist noch eine mit "Reservierung" bezeichnete Software-Taste dargestellt, die bisher nicht betrachtet worden ist. Durch Anklicken dieser Software-Taste lässt sich ein beliebiger freier Kanal der Kanäle 1 bis 8 in dem Visualisierungsprogramm gemäß der Erfindung durch einen Benutzer für ein bestimmtes zu trocknendes Endoskop reservieren und somit der freien Verfügbarkeit für die Zuordnung zu anderen zu trocknendes Endoskopen entziehen.

Abschließend sei noch angemerkt, dass die Erfindung auf den Einsatz eines normalen Personalcomputers für die erläuterten Visualisierungen nicht beschränkt ist. Vielmehr kommt auch jegliche andere Visualisierungsvorrichtung in Betracht, bei der als Eingabevorrichtung gegebenenfalls eine virtuelle Softwaretastatur verwendet wird.

## Patentansprüche

1. Verfahren zum Visualisieren von Trocknungsvorgängen von in einem Trockenschrank untergebrachten Endoskopen nach deren Reinigung und/oder Desinfektion, **dadurch gekennzeichnet, dass** in einer Anzeige-Bildschirmmaske eines Visualisierungsprogramms den einzelnen Endoskopen zugeordnete schematische Endoskopdarstellungen angezeigt werden, die bei fortschreitender Trocknung der entsprechenden Endoskope in einer Weise verändert werden, insbesondere durch farbliche Füllung und/oder eine Formveränderung, die vom Ausgangszustand der Trocknung verschieden ist.

2. Verfahren nach Anspruch 1, **dadurch gekennzeichnet, dass** die Anzeige-Bildschirmmaske als Hintergrundmaske oder als Bildschirmschoner in einer Anzeigeeinrichtung benutzt wird.

3. Verfahren nach Anspruch 1 oder 2, **dadurch gekennzeichnet, dass** das Trocknen betreffende Parameter als Einstellgrößen, insbesondere als Trocknungstemperatur und Trocknungsluftgeschwindigkeit, mittels den einzelnen Endoskopen bzw. deren zugehörigen schematischen Endoskopdarstellungen zugehöriger Software-Tasten individuell eingestellt werden, die insbesondere in einer gesonderten Bildschirmmaske dargestellt werden.

4. Verfahren nach einem der Ansprüche 1 bis 3, **dadurch gekennzeichnet, dass** den einzelnen Endoskopdarstellungen gegebenenfalls in einer weiteren Bildschirmmaske Anzeigebereiche zugeordnet werden, in denen Werte der durch das jeweilige Endoskop hindurchgeleitete Trocknungsluft pro Zeiteinheit und die Resttrocknungszeit angezeigt werden.

5. Verfahren nach einem der Ansprüche 1 bis 4, **dadurch gekennzeichnet, dass** durch ein tastenbetätigtes Auswahlmenü der jeweiligen Endoskopdarstellung eine dem entsprechenden Endoskop zugehörige Geräte- und/oder Typenbezeichnung zugeordnet und angezeigt wird.

6. Verfahren nach einem der Ansprüche 1 bis 5, **dadurch gekennzeichnet, dass** sämtliche Darstellungen bzw. Anzeigen durch Anklicken wenigstens einer Software-Taste in einer Speichervorrichtung für eine Protokollierung der Trocknungsvorgänge sowie für die Erstellung von Diagrammen gespeichert werden.

7. Vorrichtung zur Visualisierung von Trocknungsvorgängen bei einer Mehrzahl von in einem Trockenschrank untergebrachten Endoskopen nach deren Reinigung und/oder Desinfektion mittels einer Visualisierungsvorrichtung, umfassend Mittel zur Ausführung des Verfahrens nach Anspruch 1,
wobei in der Visualisierungsvorrichtung eine Anzelge-Bildschlrmaske mit schematischen Endoskopdarstellungen der zu trocknenden Endoskope anzeigbar ist und wobei die schematischen Endoskopdarstellungen der zu trocknenden Endoskope in der Anzeige-Bildschirmmaske bei der fortschreitenden Trocknung der betreffenden Endoskope in einer Weise verändert sind, insbesondere durch färbliche Füllung und/oder eine Formveränderung, welche vom Ausgangszustand der Trocknung der Endoskope verschieden ist.

8. Vorrichtung nach Anspruch 7, **dadurch gekennzeichnet, dass** die Anzeige-Bildschirmmaske als Hintergrundmaske oder als Bildschirmschoner in einer Anzeigeeinrichtung nutzbar ist.

9. Vorrichtung nach Anspruch 7 oder 8, **dadurch gekennzeichnet, dass** das Trocknen betreffende Parameter als Einstellgrößen, insbesondere als Trocknungstemperatur und/oder Trocknungsluftgeschwindigkeit, mittels den einzelnen Endoskopen bzw. deren zugehörigen schematischen Endoskopdarstellungen zugehöriger Software-Tasten individuell einstellbar sind, die insbesondere in einer gesonderten Bildschirmmaske enthalten sind.

10. Vorrichtung nach einem der Ansprüche 7 bis 9, **dadurch gekennzeichnet, dass** den einzelnen Endoskopdarstellungen gegebenenfalls in einer weiteren Bildschirmmaske Anzeigebereiche zugeordnet sind, in denen Werte der durch das jeweilige Endoskop hindurchgeleitete Trocknungsluft pro Zeiteinheit und/oder die Resttrocknungszeit anzeigbar sind.

11. Vorrichtung nach einem der Ansprüche 7 bis 10, **dadurch gekennzeichnet,dass** durch ein tastenbetätigtes Auswahlmenü der jeweiligen Endoskopdarstellung eine dem entsprechenden Endoskop zugehörige Geräte- und/oder Typenbezeichnung zuzuordnen und anzuzeigen ist.

12. Vorrichtung nach einem der Ansprüche 7 bis 11, **dadurch gekennzeichnet,dass** eine Speichervorrichtung vorgesehen ist, in der sämtliche Darstellungen und/oder Anzeigewerte durch Anklicken wenigstens einer Software-Taste für eine Protokollierung der Trocknungsvorgänge sowie für die Erstellung von Diagrammen speicherbar sind.

13. Computerprogramm zum Betreiben einer das Verfahren nach einem der Ansprüche 1 bis 6 ausführenden Vorrichtung nach einem der Ansprüche 7 bis 12.

## Claims

1. Method for visualizing the drying of endoscopes housed in a drying cabinet after the cleaning and/or disinfection of said endoscopes, **characterized in that** schematic endoscope representations assigned to the individual endoscopes are displayed in a display screen mask of a visualization program, which endoscope representations are somehow modified, in particular by a filling in terms of colour and/or a shape change which differs from the initial state of the drying, while the drying of the corresponding endoscopes progresses.

2. Method according to Claim 1, **characterized in that** the display screen mask is used as background mask or as screensaver in a display apparatus.

3. Method according to Claim 1 or 2, **characterized in that** parameters relating to the drying are set individually as setting variables, in particular as drying temperature and drying air velocity, by means of the software keys belonging to the individual endoscopes or the associated schematic endoscope representations thereof, said software keys more particularly being displayed in a separate screen mask.

4. Method according to one of Claims 1 to 3, **characterized in that** display areas, which are optionally in a further screen mask, are associated with the individual endoscope representations, with values of the drying air per unit time conveyed through the respective endoscope and the remaining drying time being displayed in said display areas.

5. Method according to one of Claims 1 to 4, **characterized in that** an instrument and/or type designation belonging to the corresponding endoscope is assigned and displayed using a key-actuated selection menu for the respective endoscope representation.

6. Method according to one of Claims 1 to 5, **characterized in that** by clicking at least one software key all representations or displays are stored in a storage device, for a log of the drying and for the creation of diagrams.

7. Device for visualizing by means of a visualization device drying in the case of a plurality of endoscopes housed in a drying cabinet after the cleaning and/or disinfection of said endoscopes, comprising means for carrying out the method according to Claim 1,
wherein a display screen mask with schematic endoscope representations of the endoscopes to be dried can be displayed in the visualization device and wherein the schematic endoscope representations of the endoscopes to be dried are somehow modified in the display screen mask, in particular by a filling in terms of colour and/or a shape change which differs from the initial state of the drying of the endoscope, while the drying of the corresponding endoscopes progresses.

8. Device according to Claim 7, **characterized in that** the display screen mask can be used as background mask or as screensaver in a display apparatus.

9. Device according to Claim 7 or 8, **characterized in that** parameters relating to the drying can be set individually as setting variables, in particular as drying temperature and/or drying air velocity, by means of the software keys belonging to the individual endoscopes or the associated schematic endoscope representations thereof, said software keys more particularly being contained in a separate screen mask.

10. Device according to one of Claims 7 to 9, **characterized in that** display areas, which are optionally in a further screen mask, are associated with the individual endoscope representations, with it being possible to display values of the drying air per unit time conveyed through the respective endoscope and/or the remaining drying time in said display areas.

11. Device according to one of Claims 7 to 10, **characterized in that** an instrument and/or type designation belonging to the corresponding endoscope is to be assigned and displayed using a key-actuated selection menu for the respective endoscope representation.

12. Device according to one of Claims 7 to 11, **characterized in that** provision is made for a storage device, in which all representations and/or display values can be stored by clicking at least one software key, for a log of the drying and for the creation of diagrams.

13. Computer program for operating a device according to one of Claims 7 to 12 which carries out the method according to one of Claims 1 to 6.

## Revendications

1. Procédé de visualisation de processus de séchage d'endoscopes placés dans une armoire de séchage après nettoyage et/ou désinfection, **caractérisé en ce que** des représentations schématiques d'endoscopes associées aux endoscopes individuels sont affichées dans un masque d'écran d'affichage d'un programme de visualisation, lesquelles représentations sont modifiées d'une certaine manière au cours du séchage des endoscopes correspondants, notamment par un remplissage coloré et/ou une modification de forme qui est/sont différents de l'état initial du séchage.

2. Procédé selon la revendication 1, **caractérisé en ce que** le masque d'écran d'affichage est utilisé en tant que masque d'arrière-plan ou en tant qu'écran de veille dans une dispositif d'affichage.

3. Procédé selon la revendication 1 ou 2, **caractérisé en ce que** des paramètres liés au séchage tels que des valeurs de réglage, notamment tels que la température de séchage et la vitesse de séchage, sont réglés individuellement au moyen de touches logicielles associées aux endoscopes individuels ou aux représentations schématiques d'endoscopes qui leur sont associées, lesquelles touches sont notamment représentées sur un masque d'écran séparé.

4. Procédé selon l'une quelconque des revendications 1 à 3, **caractérisé en ce qu'**on affecte aux représentations d'endoscopes individuelles, le cas échéant dans un masque d'écran supplémentaire, des zones d'affichage dans lesquelles on affiche des valeurs du débit d'air de séchage acheminé à travers l'endoscope respectif par unité de temps et le temps de séchage résiduel.

5. Procédé selon l'une quelconque des revendications 1 à 4, **caractérisé en ce qu'**une désignation d'appareil et/ou de type associée à l'endoscope correspondant est affectée à la représentation d'endoscope respective et est affichée au moyen d'un menu de sélection par actionnement de touches.

6. Procédé selon l'une quelconque des revendications 1 à 5, **caractérisé en ce que** la totalité des représentations ou des affichages sont stockés dans un dispositif de mémoire en cliquant sur au moins une touche logicielle pour une journalisation des processus de séchage ainsi que pour la création de diagrammes.

7. Dispositif de visualisation, au moyen d'un dispositif de visualisation, de processus de séchage d'une pluralité d'endoscopes placés dans une armoire de séchage après leur nettoyage et/ou désinfection, comprenant des moyens destinés à mettre en oeuvre le procédé selon la revendication 1,
dans lequel un masque d'écran d'affichage comportant des représentations schématiques d'endoscopes des endoscopes à sécher peut être affiché dans le dispositif de visualisation et dans lequel les représentations schématiques d'endoscopes des endoscopes à sécher sont modifiées d'une certaine manière dans le masque d'écran d'affichage au cours du séchage des endoscopes concernés, notamment par remplissage coloré et/ou une modification de forme qui est/sont différents de l'état initial du séchage des endoscopes.

8. Dispositif selon la revendication 7, **caractérisé en ce que** le masque d'écran d'affichage peut être utilisé en tant que masque d'arrière-plan ou en tant qu'écran de veille dans une dispositif d'affichage.

9. Dispositif selon la revendication 7 ou 8, **caractérisé en ce que** des paramètres liés au séchage tels que des valeurs de réglage, notamment tels que la température de séchage et la vitesse de séchage, peuvent être réglés individuellement au moyen de touches logicielles associées aux endoscopes individuels ou aux représentations schématiques d'endoscopes qui leur sont associées, lesquelles touches sont notamment contenues dans un masque d'écran séparé.

10. Dispositif selon l'une quelconque des revendications 7 à 9, **caractérisé en ce qu'**on affecte aux représentations d'endoscopes individuelles, le cas échéant dans un masque d'écran supplémentaire, des zones d'affichage dans lesquelles on peut afficher des valeurs du débit d'air de séchage acheminé à travers l'endoscope respectif par unité de temps et/ou le temps de séchage résiduel.

11. Dispositif selon l'une quelconque des revendications 7 à 10, **caractérisé en ce qu'**une désignation d'appareil et/ou de type associée à l'endoscope correspondant est affectée à la représentation d'endoscope respective et est affichée au moyen d'un menu de sélection par actionnement de touches.

12. Dispositif selon l'une quelconque des revendications 7 à 11, **caractérisé en ce qu'**il est prévu un dispositif de mémoire dans lequel la totalité des représentations et/ou des affichages peuvent être stockés en cliquant sur au moins une touche logicielle dans un dispositif de mémoire pour une journalisation des processus de séchage ainsi que pour la création de diagrammes.

13. Programme informatique pour faire fonctionner un dispositif selon l'une quelconque des revendications 7 à 12 mettant en oeuvre le procédé selon l'une quelconque des revendications 1 à 6.
